# EUROPÄISCHE PATENTANMELDUNG

(11) **EP 1 495 732 A1**
(43) Veröffentlichungstag der Anmeldung: **12.01.2005**
(21) Anmeldenummer: 04022612.8
(22) Anmeldetag: 14.02.2000
(51) Int. Cl.: A61B 17/72

(54) **Aktiver Marknagel zur Distraktion von Knochenteilen**

(30) Priorität: 16.02.1999 DE 19906423
(62) Teilanmeldung aus: 00905064.2
(71) Anmelder: Wittenstein AG, 97999 Igersheim (DE)
(72) Erfinder: Stauch, Roman, 97959 Assamstadt (DE); Klein, Jürgen, 97990 Weikersheim (DE)
(74) Vertreter: Weiss, Peter, Dr.

(57) **Zusammenfassung**

Aktiver Marknagel zur Distraktion von Knochenteilen aus zwei gegeneinander bewegbaren Elementen, Prothese od. dgl., mit mindestens einem elektrisch betriebenen Antriebselement, wobei der Marknagel als Prothese ausgebildet ist.

## Beschreibung

Die vorliegende Erfindung betrifft einen aktiven Marknagel zur Distraktion von Knochensegmenten aus zwei gegeneinander bewegbaren Elementen, Prothese od. dgl. mit zumindest einem elektrisch betriebenen Antriebselement.

Derartige Marknägel sind in vielfältigster Form und Ausführungen auf dem Markt bekannt und gebräuchlich. Immer häufiger werden elektrische Antriebselemente in den Marknägeln vorgesehen, um eine Distraktion zweier gegeneinander bewegbarer Elemente zu erzeugen.

Der vorliegenden Erfindung liegt die Aufgabe zugrunde, einen aktiven Marknagel zur Distraktion zu schaffen, welcher platzsparend und universell einzusetzen ist.

Zur Lösung dieser Aufgabe führt, dass der Marknagel als Prothese ausgebildet ist.

Von Vorteil ist bei der vorliegenden Erfindung sollte der Marknagel als Prothese ausgebildet sein, dass nach einer gewünschten Distraktion lediglich die elektrische Steckverbindung operativ herausgelöst wird. Eine Öffnung kann dann mit einem Propfen od. dgl. verschlossen werden. Sollte eine weitere Distraktion erforderlich sein, so kann operativ der Propfen wieder entfernt und dann der Stecker dort eingesteckt werden. Dies schafft erhebliche Vorteile insbesondere beim Operieren und Handhaben derartiger aktiver Marknägel.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine schematisch dargestellte Ansicht auf einen zumindest teilweise aufgeschnittenen aktiven Marknagel;
Figur 2a einen zumindest teilweisen Längsschnitt durch den Marknagel gemäss Figur 1;
Figur 2b einen Querschnitt durch den Marknagel entlang Linie II-II gemäss Figur 2a;
Figur 3a einen Teillängsschnitt durch den Marknagel gemäss Figur 1 als weiteres Ausführungsbeispiel;
Figur 3b einen Querschnitt durch den Marknagel gemäss Figur 3a entlang Linie III-III;
Figur 4 einen zumindest teilweise dargestellten Teillängsschnitt durch ein weiteres Ausführungsbeispiel eines Marknagels gemäss Figur 1.

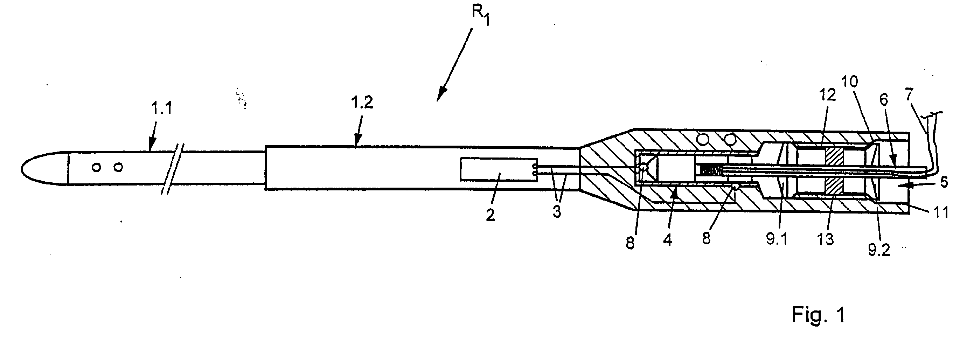

Gemäss Figur 1 weist ein erfindungsgemässer aktiver Marknagel R₁ zwei axial gegeneinander bewegbare Elemente 1.1, 1.2 auf. Im bevorzugten Ausführungsbeispiel ist das Element 1.1 gegenüber dem Element 1.2 verfahrbar. Hier nicht dargestellte Antriebsmittel, wie bspw. Getriebe, Schubstangen, Formgedächtnisantriebe od. dgl. können das Element 1.1 gegenüber dem Element 1.2 bewegen. Vorzugsweise ist im Element 1.2 des Marknagels R₁ zumindest ein elektrisches Antriebselement 2 vorgesehen. Dieses steht über elektrische Verbindungsleitungen 3 mit einer Buchse 4 des Elementes 1.2 in Verbindung. Die Buchse 4 ist stirn- und endseitig des Elementes 1.2 im Marknagel vorgesehen. Durch eine Öffnung 5 lässt sich ein Steckerelement 6 in die Buchse 4 einschieben. An das Steckerelement 6 schliessen Energieversorgungsleitungen 7 an, welche bspw. zu einer externen Energiequelle führen. Von dort kann.Energie direkt oder indirekt bspw. induktiv eingeleitet werden. Diese Energie ist zum Betreiben und Steuern des Antriebselementes 2 erforderlich.

Damit über einen gewissen Zeitraum, während der aktive Marknagel R₁ im Knochen verbleibt, eine gewünschte Distraktion erfolgen kann, ist es erforderlich eine elektrische Verbindung permanent herzustellen. Dabei hat sich als besonders vorteilhaft erwiesen stirnseitig, insbesondere endseitig in das Element 1.2 ein sogenanntes Steckerelement 6 einzustecken. Entsprechende Kontaktstellen 8 übertragen elektrische Energie und/oder elektrische Signale.

Wichtig bei derartigen Verbindungen ist jedoch, dass aus hygienischen Gründen eine absolute dichte saubere wiederlösbare Verbindungsstelle zwischen Marknagel R₁ und Steckerelement 6 hergestellt wird. Hierzu wird zumindest ein Dichtlippenelement 9.1, 9.2 dem Steckerelement 6 zugeordnet, welches die Öffnung 5 und damit die Buchse 4 des Elementes 1.2 wiederlösbar verschliesst. Hierdurch bleibt gewährleistet, dass über lange Zeiträume immer ein elektrischer Kontakt wiederlösbar hergestellt bleibt.

Ausserdem gewährleistet das äussere Dichtlippenelement 9.2 einen optimalen Knickschutz, welcher bei den vorhandenen Biegewechselbelastungen von Bedeutung ist.

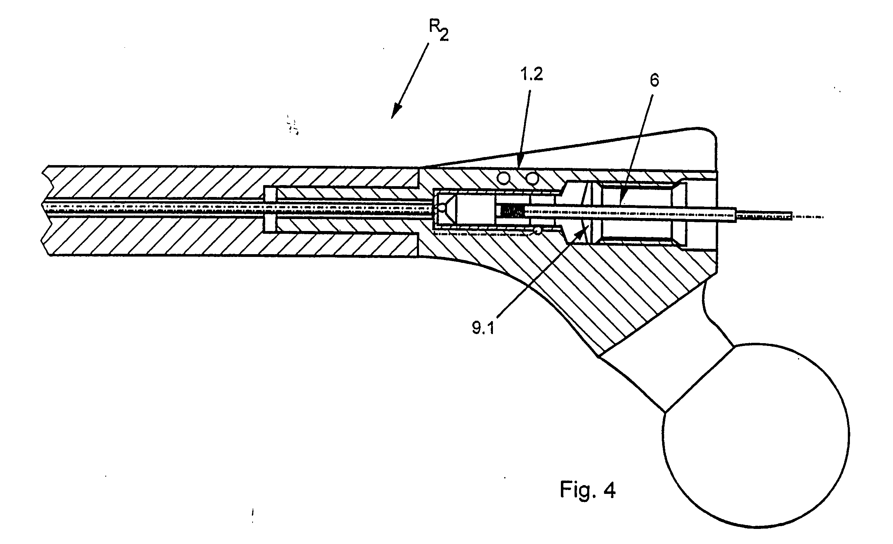

Ist bspw. der aktive Marknagel eine Hüftgelenksendprothese, wie sie in Figur 4 teilweise dargestellt ist, oder eine Kniegelenksendprothese, so kann diese ebenfalls, wie oben beschrieben, mit einer entsprechenden Steckverbindung vorgesehen sein. Hierdurch lässt sich bspw. nach der Operation durch eine entsprechende Distraktion eine Korrektur vornehmen. Dies kann über einen längeren Zeitraum der Genesung geschehen. Ist dann bspw. keine Korrektur mehr erforderlich so wird lediglich das Steckerelement aus der Öffnung 5 des Elementes 1.2 entfernt. Gegebenenfalls wird die Öffnung 5 mit einem dichten Propfenverschluss od. dgl. verschlossen, sollte nochmals ein Korrektur erforderlich sein. Auf diese Weise lassen sich als aktive Marknägel R₁ ausgebildete Prothesen permanent bedienen, wobei nach einer entsprechenden Distraktion oder Korrektur des Knochens ein Entfernen der elektrischen Anschlüsse ohne weiteres möglich ist.

Sollte nach einer bestimmten Dauer eine weitere Korrektur erforderlich werden, so kann das Steckerelement wieder in die Öffnung eingeführt werden. Eine ggfs. vollimplantierbare subkutane Energiequelle, an welche Engergie oder entsprechende Signale induktiv weitergegeben werden, kann dann unter der Haut implantiert werden. Der aktive Marknagel kann auf diese Weise wieder in Betrieb genommen werden.

Von Bedeutung ist auch ein radiales Dichtlippenelement 9.1, 9.2, welches ggfs. an einem nach innen ragenden Absatz 10 anliegt. Bevorzugt ist das Dichtlippenelement 9.1, 9.2 nach aussen verjüngt ausgebildet und liegt dicht an einer Innenwand 11 der Buchse 4 bzw. des Elementes 1.2 an.

Es ist ferner daran gedacht ein Innengewinde 12 innerhalb der Buchse 4 vorzusehen, in welche ein entsprechendes Aussengewinde 13 des Steckerelementes 6 eingreift, um das Steckerelement 6 wiederlösbar festzulegen. Bevorzugt werden jedoch Rastverbindungen od. dgl. verwendet.

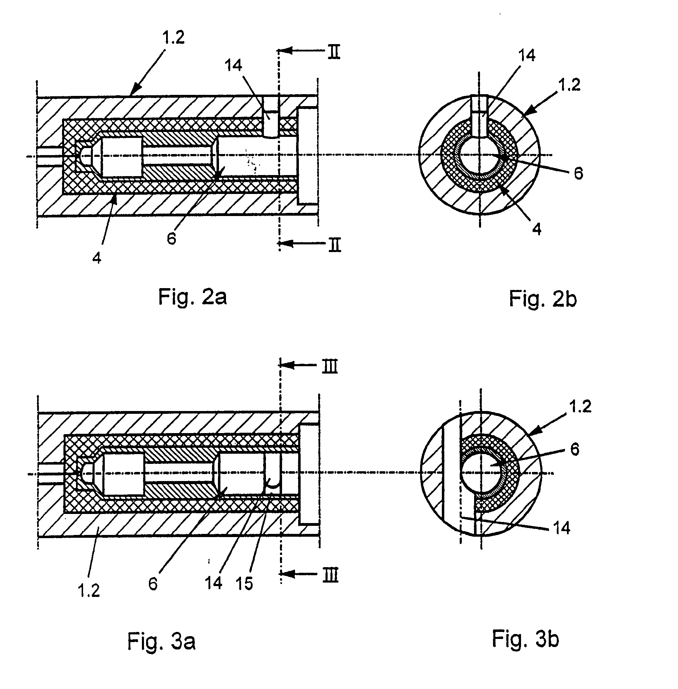

Als Sicherung gegen ein Herausziehen im Betrieb können einfache Gewindestifte od. dgl. vorgesehen sein, wie sie bspw. in Figur 2a aufgezeigt sind. Dort durchgreift ein entsprechendes Sicherungselement 14 als Gewindestift radial das Element 1.2, um das in der Buchse 4 eingesteckte Steckerelement 6 wiederlösbar festzulegen bzw. festzuklemmen. Dies wird auch in der querschnittlichen Darstellung gemäss Figur 2b verdeutlicht.

Ein weiteres Sicherungselement 14 zeigt die Figuren 3a und 3b, welches in eine entsprechende Nut 15 des Steckerelementes 6 eingreift und hierdurch eine axiale Sicherung des Steckerelementes 6 gegenüber der Buchse 4 des Elementes 1.2 bildet. Dabei kann das Sicherungselement 14 als Riegel od. dgl. vorgesehen sein, um ein axiales Bewegen des Steckerelementes 6 gegenüber der Buchse 4 zu verhindern. Hier sei der Erfindung keine Grenze gesetzt.

In dem letzten Ausführungsbeispiel gemäss Figur 4 ist verdeutlicht, dass auch in beliebige Prothesen, Hüftgelenksendprothesen als aktive Marknägel R₂ entsprechende Steckerelemente 6, in oben beschriebener Weise, eingesetzt sein können. Dies soll auch im Rahmen der vorliegenden Erfindung liegen.

| **Positionszahlenliste** | | | | | |
|---|---|---|---|---|---|
| 1 | Element | 34 | | 67 | |
| 2 | Antriebselement | 35 | | 68 | |
| 3 | Verbindungsleitung | 36 | | 69 | |
| 4 | Buchse | 37 | | 70 | |
| 5 | Öffnung | 38 | | 71 | |
| 6 | Steckerelement | 39 | | 72 | |
| 7 | Verbindungsleitung | 40 | | 73 | |
| 8 | Kontaktstelle | 41 | | 74 | |
| 9 | Dichtlippenelement | 42 | | 75 | |
| 10 | Absatz | 43 | | 76 | |
| 11 | Innenwand | 44 | | 77 | |
| 12 | Innengewinde | 45 | | 78 | |
| 13 | Aussengewinde | 46 | | 79 | |
| 14 | Sicherungselement | 47 | | | |
| 15 | Nut | 48 | | R₁ | aktiver Marknagel |
| 16 | | 49 | | R₂ | aktiver Marknagel |
| 17 | | 50 | | | |
| 18 | | 51 | | | |
| 19 | | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Aktiver Marknagel zur Distraktion von Knochensegmenten aus zwei gegeneinander bewegbaren Elementen (1), Prothese od. dgl., mit zumindest einem elektrisch betriebenen Antriebselement (2),
**dadurch gekennzeichnet,**
**dass** der Marknagel als Prothese ausgebildet ist.

2. Aktiver Marknagel zur Distraktion von Knochensegmenten aus zwei gegeneinander bewegbaren Elementen (1), Prothese od. dgl., mit zumindest einem elektrisch betriebenen Antriebselement (2), **dadurch gekennzeichnet, dass** der Marknagel als Hüftgelenksprothese ausgebildet ist.

3. Aktiver Marknagel nach Anspruch 1 oder 2, **dadurch gekennzeichnet, dass** endseits der aktive Marknagel als Prothese, insbesondere Hüftgelenksendprothese ausgebildet ist.

4. Aktiver Marknagel nach wenigstens einem der Ansprüche 1 bis 3, **dadurch gekennzeichnet, dass** das wiederlösbare Steckerelement (6) stirnseitig in eine Öffnung (5) des Marknagels einsetzbar ist.

5. Aktiver Marknagel nach Anspruch 4, **dadurch gekennzeichnet, dass** das Steckerelement (6) in eine Buchse (4) des Marknagels wiederlösbar eingreift und hierdurch zumindest eine elektrische Kontaktstelle (8) bildet.

6. Aktiver Marknagel nach Anspruch 4 oder 5, **dadurch gekennzeichnet, dass** dem Marknagel elektrische Energie über ein Steckerelement (6) zuführbar und an dieses eine Energiequelle, insbesondere eine induktive vollimplantierbare Energiequelle zur induktiven Energieübertragung ggfs. subkutan anschliessbar ist.
